(19) Europäisches Patentamt
European Patent Office
Office européen des brevets

(11) Numéro de publication : **0 351 336 B1**

# (12) FASCICULE DE BREVET EUROPEEN

(45) Date de publication du fascicule du brevet : **28.10.92 Bulletin 92/44**

(51) Int. Cl.$^5$ : **C07C 45/49**, C07C 47/58, C07C 47/565, C07C 47/575, C07C 47/57

(21) Numéro de dépôt : **89420251.4**

(22) Date de dépôt : **11.07.89**

## (54) Procédé de préparation d'hydroxybenzaldéhydes par hydrocarbonylation.

(30) Priorité : **13.07.88 FR 8809765**

(43) Date de publication de la demande : **17.01.90 Bulletin 90/03**

(45) Mention de la délivrance du brevet : **28.10.92 Bulletin 92/44**

(84) Etats contractants désignés : **AT BE CH DE ES FR GB GR IT LI LU NL SE**

(56) Documents cités :
**EP-A- 0 109 606**
**EP-A- 0 244 328**
**US-A- 3 960 932**

(73) Titulaire : **RHONE POULENC CHIMIE**
**25 Quai Paul Doumer**
**F-92408 Courbevoie Cédex (FR)**

(72) Inventeur : **Fompeyrine, Patricia**
**5, avenue Valioud**
**F-69110 Ste-Foy-Les-Lyon (FR)**
Inventeur : **Metz, François**
**22, rue de Condé**
**F-69002 Lyon (FR)**

(74) Mandataire : **Dutruc-Rosset, Marie-Claude et al**
**RHONE-POULENC CHIMIE Direction des Brevets 25, Quai Paul Doumer**
**F-92408 Courbevoie Cédex (FR)**

**EP 0 351 336 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a trait à un procédé de préparation d'hydroxybenzaldéhydes par hydrocarbonylation des halogénophénols correspondants.

Le brevet US 3 960 932 décrit un procédé général de préparation d'aldéhydes, par réaction d'halogénures d'aryle, de vinyle ou de composés hétérocycliques, avec un mélange de monoxyde de carbone et d'hydrogène, en présence d'une amine tertiaire et d'un catalyseur au palladium consistant en un complexe d'un dérivé du palladium divalent avec une phosphine, un phosphite ou une arsine ou en l'association d'un sel de palladium divalent ou de palladium métallique finement divisé avec un agent complexant du groupe des phosphines, des phosphites ou des arsines. Les halogénures d'aryles utilisés dans le procédé selon US 3 960 932 sont des bromures ou des iodures de phényle ou de naphtyle, non substitués ou substitués par des groupements alkyle, alcoxy, nitrile ou carboxylate d'alkyle.

Le brevet européen EP 109 606 propose d'augmenter la cinétique de la réaction d'hydrocarbonylation du précédent procédé, en opérant sous des pressions de 2 à 40 MPa (20 à 400 bar), à des températures de 80°C à 250°C et en utilisant des quantités importantes de phosphine ou de phosphite (2 à $10^5$ fois la quantité molaire de catalyseur).

Il faut observer que ces procédés de l'art antérieur ne s'appliquent pas à l'hydrocarbonylation d'halogénophénols.

C'est précisément un objet de la présente invention de fournir un procédé de préparation d'hydroxybenzaldéhydes par réaction d'un halogénophénol avec un mélange monoxyde de carbone/hydrogène, en présence d'une amine tertiaire, d'un catalyseur à base d'un métal noble et d'une phosphine.

Plus particulièrement, il s'agit d'un procédé de préparation d'un hydroxybenzaldéhyde de formule générale (I) :

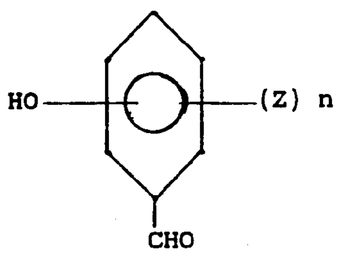

(I)

dans laquelle
- n représente 0, 1 ou 2
- Z représente un groupement électron-donneur ou un groupement électron-attracteur

par réaction d'un halogénophénol de formule générale (II)

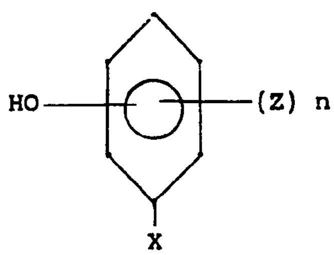

(II)

dans laquelle
- X représente un atome de brome ou un atome d'iode ;
- Z a les significations indiquées précédemment ;
- n représente 0, 1 ou 2 ;

avec un mélange monoxyde de carbone/hydrogène, en présence d'un catalyseur à base d'un métal noble, d'une amine tertiaire et d'une phosphine, caractérisé en ce que l'amine tertiaire est telle que le pKa de son acide conjugué soit supérieur au pKa de l'halogénophénol de formule (II) et, en ce que le pKa de la phosphine soit supérieur ou égal à 5.

Le pKa dans l'eau (généralement à 25°C) de l'acide conjugué de l'amine tertiaire est indiqué dans des tables que l'on trouve dans la littérature.

Le pKa dans l'eau de la phosphine est également indiqué dans des tables que l'on trouve dans la littérature.

Le pKa de l'halogénophénol de formule (II) est déterminé selon la méthode E3bg de l'IUPAC intitulée "IONISATION CONSTANTS OF ORGANIC ACIDS IN AQUEOUS SOLUTIONS" (éditeur Pergamon Press ; 1979).

Il a donc été trouvé que pour pouvoir hydrocarbonyler un halogénophénol de formule (II) afin de préparer un hydroxybenzaldéhyde de formule (I), d'une part le pKa de l'acide conjugué de l'amine tertiaire utilisée doit être supérieur ou égal à celui de l'halogénophénol (II) mis en oeuvre et, d'autre part, le pKa de la phosphine doit être supérieur ou égal à 5.

Lorsque l'amine tertiaire ou la phosphine utilisées ne répondent pas à ces conditions, il y a essentiellement formation d'un composé polymérique, mais pratiquement pas d'hydroxybenzaldéhyde.

Les halogénophénols de formule (II) auxquels s'applique la présente invention sont plus particulièrement ceux pour lesquels le symbole Z représente un radical hydroxyle, un atome de brome, un atome d'iode, un radical alkyle, un radical alcoxy, un radical alkyle ou alcoxy substitué par un ou plusieurs atomes de chlore ou de fluor, un radical cycloalkyle, un radical phényle, un radical cycloalkoxy, un radical phénoxy, un radical alcoxycarbonyle, un radical cycloalcoxycarbonyle, un radical phénoxycarbonyle, un radical alkylcarbonyloxy, un radical cycloalkylcarbonyloxy, un radical phénylcarbonyloxy, l'un des radicaux précédents substitué par un ou plusieurs atomes de fluor et/ou de chlore ou groupes nitrile et le symbole X représente un atome de brome ou un atome d'iode.

Plus particulièrement dans les formules (I) et (II),

- X représente un atome de brome,
- Z représente :
  . un radical hydroxyle ;
  . un atome de brome ;
  . un radical alkyle linéaire ou ramifié de 1 à 20 atomes de carbone ou un radical alkyle substitué par un ou plusieurs atomes de fluor et/ou de chlore, tels que par exemple les radicaux méthyle, éthyle, propyles, butyles, pentyles, hexyles, octyles, décyles, trifluorométhyle, difluorochlorométhyle, trichlorométhyle ; de préférence un radical alkyle inférieur, c'est-à-dire ayant 1 à 4 atomes de carbone ou un radical alkyle inférieur substitué par 1 à 3 atomes de fluor et/ou de chlore ;
  . un radical alkoxy linéaire ou ramifié ayant 1 à 20 atomes de carbone ou un radical alcoxy substitué par un ou plusieurs atomes de fluor et/ou de chlore ; de préférence un radical alcoxy inférieur (ayant 1 à 4 atomes de carbone) ou un radical alcoxy inférieur substitué par 1 à 3 atomes de fluor et/ou de chlore, tel que par exemple les radicaux méthoxy, éthoxy, isopropoxy, difluorochlorométhoxy ou trichlorométhoxy ;
  . un radical cyclopentyle, cyclohexyle ou cyclooctyle ;
  . un radical phényle ou un radical phényle substitué par 1 à 3 radicaux alkyles ou alcoxy inférieurs, tel que les radicaux xylyles, toluyles, méthoxyphényles, éthoxyphényles ;
  . un radical alcoxycarbonyle ayant 2 à 11 atomes de carbone et de préférence 2 à 5 atomes de carbone tels que par exemple les radicaux méthoxycarbonyle, éthoxycarbonyle, propoxycarbonyle et butoxycarbonyle ;
  . un radical alcoxycarbonylalkyle dont la partie alcoxycarbonyle est telle que définie précédemment et la partie alkyle comporte 1 à 4 atomes de carbone ;
  . un radical cyclopentyloxycarbonyle ou cyclohexyloxycarbonyle ;
  . un radical phénoxycarbonyle ou méthylphénoxycarbonyle ;
  . un radical alkylcarbonyloxy ayant 2 à 11 atomes de carbone et de préférence 2 à 5 atomes de carbone, tel que par exemple les radicaux acétoxy, propionyloxy, butyryloxy ;
  . un radical cyclopentanoyloxy ou cyclohexanoyloxy ;
  . un radical benzoyloxy, méthylbenzoyloxy ou diméthylbenzoyloxy.

Dans la formule (I), Z peut en outre représenter un groupement aldéhyde.

Comme exemples spécifiques d'hydroxybenzaldéhydes de formule (I) que l'on peut obtenir par le procédé selon l'invention, on peut citer de manière non limitative l'hydroxy-4 benzaldéhyde, l'hydroxy-2 benzaldéhyde, la vanilline (ou hydroxy-4 méthoxy-3 benzaldéhyde), l'hydroxy-2 méthoxy-5 benzaldéhyde, l'hydroxy-2 méthoxy-3 benzaldéhyde, l'hydroxy-4 éthoxy-3 benzaldéhyde, l'hydroxy-2 éthoxy-3 benzaldéhyde, l'hydroxy-2 éthoxy-5 benzaldéhyde, le diméthoxy-3,5 hydroxy-4 benzaldéhyde, le dihydroxy-3,4 benzaldéhyde, le dihydroxy-2,5 benzaldéhyde, le dihydroxy-2,3 benzaldéhyde, le dibromo-3,5 hydroxy-4 benzaldéhyde, le bromo-3 hydroxy-4 benzaldéhyde, le formyl-3 hydroxy-4 benzaldéhyde.

Comme exemples spécifiques d'halogénophénols de formule (II) que l'on peut mettre en oeuvre dans le procédé selon l'invention, on peut citer non limitativement le bromo-4 phénol,le bromo-2 phénol, le bromo-4 méthoxy-2 phénol, le bromo-2 méthoxy-4 phénol, le bromo-6 méthoxy-2 phénol, le bromo-4 éthoxy-2 phénol,

le bromo-2 éthoxy-4 phénol, le bromo-6 éthoxy-2 phénol, le bromo-4 diméthoxy-2,6 phénol, le bromo-4 dihydroxy-1,2 benzène, le bromo-2 dihydroxy-1,4 benzène, le bromo-3 dihydroxy-1,2 benzène, le dibromo-2,4 phénol, le tribromo-2,4,6 phénol.

A titre de catalyseurs, on peut utiliser pour la mise en oeuvre du procédé selon l'invention, un métal noble finement divisé, du groupe VIII de la classification périodique des éléments tel que le palladium, le rhodium et l'iridium, ou leurs sels d'acides minéraux ou organiques.

Les dérivés du palladium conviennent tout particulièrement bien au procédé de l'invention.

Comme exemples spécifiques de dérivés du palladium, on peut citer les carboxylates tels que notamment les acétates, propionates, butyrates ou benzoates de palladium (II), le chlorure palladeux.

On peut également utiliser des complexes des sels minéraux ou organiques du palladium avec la phosphine.

Dans ce dernier cas, ce complexe est généralement réalisé in situ entre le dérivé du palladium et la phosphine présente. Mais on peut également préparer ledit complexe extemporanément et l'introduire dans le milieu réactionnel. On peut alors rajouter ou non une quantité supplémentaire de phosphine libre.

La quantité de catalyseur exprimée en moles d'atomes de métal ou en moles de dérivé métallique par mole d'halogènophénol de formule (I) peut varier dans de larges limites.

Ainsi, elle peut être comprise entre $10^{-5}$ et $10^{-1}$ mole/mole et de préférence entre $10^{-4}$ et $10^{-2}$ mole/mole.

La quantité de phosphine libre et/ou sous forme de complexe avec le catalyseur, est telle que le rapport molaire phosphine/métal noble du catalyseur soit au moins égal à 2.

Le rapport phosphine/métal noble peut atteindre des valeurs aussi élevées que 10.000.

Un rapport phosphine/métal noble compris entre 4 et 1.000 est en général très convenable.

Les phosphines dont le pKa est égal ou supérieur à 5 sont de manière générale des phosphines aliphatiques, cycloaliphatiques ou arylaliphatiques.

On peut aussi utiliser des phosphines mixtes, aliphatiques et/ou cycloaliphatiques et/ou arylaliphatiques et/ou aromatiques.

Ces phosphines sont notamment celles qui répondent à la formule générale (III) :

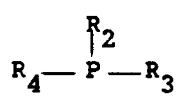

(III)

dans laquelle les symboles $R_2$, $R_3$ et $R_4$, identiques ou différents, représentent :

- un radical alkyle ayant de 1 à 12 atomes de carbone,
- un radical cycloalkyle ayant de 5 ou 6 atomes de carbone,
- un radical cycloalkyle ayant de 5 ou 6 atomes de carbone, substitué par un ou plusieurs radicaux alkyles ayant 1 à 4 atomes de carbone, alkoxy ayant 1 à 4 atomes de carbone,
- un radical phénylalkyle dont la partie aliphatique comporte de 1 à 6 atomes de carbone,
- un ou deux des radicaux $R_2$, $R_3$ et $R_4$ représentent un radical phényle ou un radical phényle substitué par un ou plusieurs radicaux alkyles ayant 1 à 4 atomes de carbone ou alkoxy ayant 1 à 4 atomes de carbone.

A titre d'exemples de telles phosphines, on peut citer non limitativement : la tricyclohexylphosphine, la triméthylphosphine, la triéthylphosphine, la tri-n-butylphosphine, la triisobutylphosphine, la tritertiobutylphosphine, la tribenzylphosphine, la dicyclohexylphénylphosphine, la diméthylphénylphosphine, la diéthylphénylphosphine, la ditertiobutylphénylphosphine.

L'amine tertiaire utilisée dans le présent procédé peut être une amine de formule générale (IV)

$$N-(R_1)_3 \qquad (IV)$$

dans laquelle :

- les radicaux $R_1$, identiques ou différents, représentent des restes hydrocarbonés ayant de 1 à 20 atomes de carbone, tels que des radicaux alkyle, cycloalkyle, aryle ou hétérocyclique ;
- 2 radicaux $R_1$ forment ensemble et avec l'atome d'azote un hétérocycle ayant 4 à 6 atomes.

Plus particulièrement :

- les symboles $R_1$ représentent un radical alkyle ayant 1 à 10 atomes de carbone et de préférence 1 à 4 atomes de carbone ou un radical cyclopentyle ou cyclohexyle ou un radical pyridinyle ;
- 2 radicaux $R_1$ forment ensemble et avec l'atome d'azote un cycle pipéridine ou pyrrolidine.

A titre d'exemples de telles amines, on peut citer la triéthylamine, la tri-n-propylamine, la tri-n-butylamine, la méthyldibutylamine, la méthyldicyclohexylamine, l'éthyldiisopropylamine, la N,N-diéthylcyclohexylamine, la diméthylamino-4 pyridine, la N-méthylpipéridine, la N-éthylpipéridine, la N-n-butylpipéridine, la diméthyl-1,2 pipéridine, la N-méthylpyrrolidine, la diméthyl-1,2 pyrrolidine.

Lorsque l'on met en oeuvre un halogénophénol comme le bromo-2 phénol, le bromo-4 phénol, le bromo-4 méthoxy-2 phénol ou le bromo-4 éthoxy-2 phénol qui conduisent à des hydroxybenzaldéhydes très importants et dont le pKa (à 25°C) est de 9,5 pour le bromo-4 phénol et le bromo-4 méthoxy-2 phénol, et proche de cette valeur pour le bromo-4 éthoxy-2 phénol et de 8,55 pour le bromo-2 phénol, les amines tertiaires de formule (IV) telles que la triéthylamine qui ont un pKa de leur acide conjugué plus élevé que 9,5, conviennent bien.

La quantité d'amine tertiaire utilisée doit être suffisante pour neutraliser l'hydracide libéré par la réaction.

En outre, la concentration en amine tertiaire dans le milieu doit être au moins de 2 moles par litre pendant la durée de la réaction.

Il n'y a pas de limite supérieure critique à la quantité d'amine tertiaire, qui peut donc être utilisée en large excès par rapport à la quantité théoriquement nécessaire à la neutralisation de l'hydracide formé.

Pour maintenir la concentration en amine tertiaire, au moins égale aux valeurs limites indiquées précédemment, pendant toute la durée de la réaction, la quantité d'amine engagée doit être calculée de telle façon qu'en fin de réaction, la concentration soit au moins égale à ces valeurs. On peut également ajouter une quantité d'amine tertiaire supplémentaire au fur et à mesure du déroulement de la réaction, afin de compenser la quantité d'amine consommée par la neutralisation de l'hydracide.

On peut utiliser des mélanges $CO/H_2$ présentant différents rapports molaires des deux gaz. Généralement, le rapport molaire $CO/H_2$ varie entre 0,1 et 10.

La pression sous laquelle est mis en oeuvre le procédé varie aussi très largement. Généralement, elle va de 0,1 à 30 MPa (1 à 300 bar) et de préférence de 1 à 15 MPa (10 à 150 bar).

Le procédé selon l'invention est conduit en phase liquide.

On peut faire appel à un solvant inerte dans les conditions de la réaction d'hydrocarbonylation. Ainsi, on peut utiliser des hydrocarbures aliphatiques ou cycloaliphatiques saturés tels que l'hexane ou le cyclohexane, ou aromatiques tels que le benzène, le toluène, les xylènes ; des esters tels que le benzoate de méthyle, le téréphtalate de méthyle, l'adipate de méthyle, le phtalate de dibutyle ; des esters ou éthers de polyols tels que le diacétate de tétraéthylène glycol ; des éthers cycliques tels que le tétrahydrofuranne ou le dioxanne.

La concentration de l'halogénophénol de formule (II) mis en oeuvre dans le solvant peut varier dans de très larges limites, jusqu'à la saturation dans les conditions opératoires. Généralement, il n'est pas économiquement intéressant d'utiliser moins de 5 % en poids d'halogénophénol par volume de solvant.

De manière générale, la concentration en poids d'halogénophénol par volume de solvant est comprise entre 5 % et 50 % et de préférence entre 10 % et 40 %.

En pratique, on peut mettre en oeuvre le procédé selon l'invention en introduisant dans un autoclave inerte l'halogénophénol de formule (II), l'amine tertiaire, le catalyseur, la phosphine et le solvant, puis, après les purges habituelles, en alimentant l'autoclave avec une pression adéquate d'un mélange $CO/H_2$ ; le contenu de l'autoclave est ensuite porté sous agitation à la température convenable jusqu'à ce que l'absorption cesse. La pression dans l'autoclave peut être maintenue constante pendant la durée de la réaction grâce à une réserve de mélange gazeux, qui l'alimente à la pression choisie.

En fin d'essai, l'autoclave est refroidi et dégazé. Le mélange réactionnel est récupéré.

Un mode de traitement très simple consiste à ajouter au mélange réactionnel une solution aqueuse d'hydroxyde de métal alcalin.

Après agitation, puis décantation, on obtient ainsi une phase aqueuse et une phase organique. La phase organique contient essentiellement le catalyseur, la phosphine et au moins une partie de l'amine tertiaire. Cette solution organique peut facilement être recyclée dans une nouvelle réaction d'hydrocarbonylation, après ajout d'une nouvelle charge de l'halogénophénol et d'un éventuel complément en amine tertiaire.

La phase aqueuse contient essentiellement l'hydroxybenzaldéhyde formé sous forme du phénate de métal alcalin, ainsi que les éventuels sous- produits et l'halogénophénol éventuellement non transformé, également sous forme de dérivés de métal alcalin.

Une simple acidification et soit une recristallisation lorsque le produit est solide, soit une distillation lorsque le produit est liquide, permettent de récupérer l'hydroxybenzaldéhyde pur.

Le procédé peut être mis en oeuvre en discontinu ou en continu comme indiqué précédemment en recyclant le catalyseur, la phosphine et l'amine tertiaire.

Les exemples qui suivent illustrent l'invention.

<u>Exemples 1 et 2 et essai comparatif A</u>

Dans un autoclave de 125 $cm^3$, en alliage de marque commerciale HASTELLOY B2, muni d'un dispositif de chauffage et d'une agitation on charge :

- 10,15 g (50 mmol) de bromo-4 méthoxy-2-phénol
- 0,22 g (1 mmol) de diacétate de palladium
- 5 mmol de phosphine (indiquée dans le tableau ci-après)
- 110 mmol de diméthylamino-4 pyridine
- 17,5 $cm^3$ de toluène.
  . pKa du bromo-4 méthoxy-2 phénol : 9,5.
  . pKa de l'acide conjugué de l'amine : 9,55

L'autoclave est fermé et purgé à l'aide d'un mélange équimoléculaire de CO et de $H_2$.

On charge ensuite 0,1 MPa (1 bar) de pression de ce mélange $CO/H_2$ ; on porte sous agitation le contenu de l'autoclave à 100°C ; on ajuste la pression $CO/H_2$ à 3 MPa (30 bar), puis la température est portée à 150°C.

On maintient la température à 150°C et la pression à 3 MPa jusqu'à la fin d'absorption du mélange $CO/H_2$.

L'autoclave est alors refroidi, dégazé.

Après prélèvement d'un échantillon pour dosage par chromatographie liquide, on ajoute au mélange réactionnel 40 $cm^3$ d'une solution aqueuse de soude (6 g de soude) et on agite l'autoclave à température ambiante pendant 1 heure.

On sépare la phase aqueuse par décantation, on l'acidifie par HCl jusqu'à pH 1 et on l'extrait par 3 fois 100 $cm^3$ d'éther éthylique.

La solution éthérée obtenue est traitée par 2 fois 50 $cm^3$ d'une solution aqueuse d'hydrogénocarbonate de sodium à 20 %.

La solution éthérée est ensuite décantée et l'éther est évaporé.

Le solide brun obtenu est recristallisé dans le toluène, puis dans l'eau pour conduire à la vanilline pure.

Le tableau (I) ci-après reprend pour chaque essai les indications concernant la phosphine, la durée, le taux de transformation (TT %) du bromo-4 méthoxy-2 phénol (BMPH), le rendement (RT %) en hydroxy-4 méthoxy-3 benzaldéhyde (vanilline) (HMBZ) par rapport au bromo-4 méthoxy-2 phénol transformé, les RT % en gaïacol et éventuellement en acide hydroxy-4 méthoxy-3 benzoïque formés.

La différence à 100 % de la somme des RT indiqués précédemment correspond à la formation d'un composé polymérique de structure :

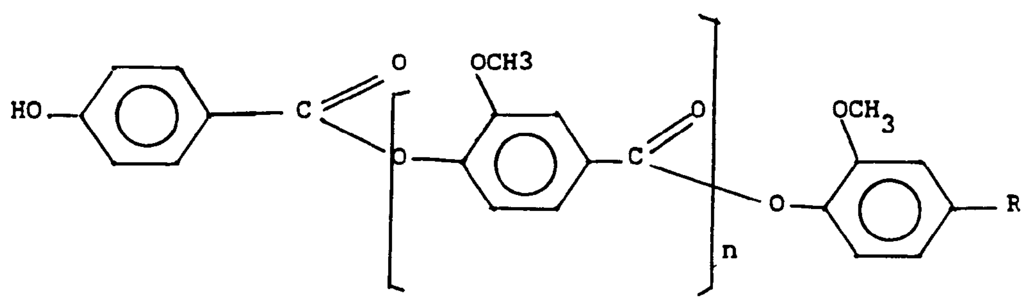

n étant égal ou supérieur à 1 et R représentant CHO, H ou Br.

On note que lorsque le pKa de la phosphine est inférieur à 5 le rendement en aldéhyde est faible (essai comparatif A).

TABLEAU I

| Essais | Phosphine | pKa * | Durée | TT % en BMPH ** | RT % en HMBZ ** | RT % en gaïacol | RT % en HMBQ ** |
|---|---|---|---|---|---|---|---|
| Exemple 1 | tricyclohexyl phosphine | 9,7 | 2 h 40 | 96 | 60,5 | 9,6 | 0 |
| Exemple 2 | triéthyl phosphine | 8,69 | 5 h 30 | 73 | 37 | 45 | 0 |
| Essai comparatif A | triphényl phosphine | 2,74 | 20 min | 100 | 8 | 2 | 0,2 |

* pka de la phosphine

** BMPH = bromo-4 méthoxy-2 phénol
HMBZ = hydroxy-4 méthoxy-3 benzaldéhyde (vanilline)
HMBQ = acide hydroxy-4 méthoxy-3 benzoïque

Exemples 3 à 5 et essai comparatif B

Dans un autoclave de 125 $cm^3$, en alliage de marque commerciale HASTELLOY B2, muni d'un dispositif de chauffage et d'une agitation on charge :

- 10,15 g (50 mmol) de bromo-4 méthoxy-2 phénol
- 0,22 g (1 mmol) de diacétate de palladium
- 5 mmol de phosphine (indiquée dans le tableau ci-aprés)
- 110 mmol de triéthylamine
- 17,5 $cm^3$ de toluène.

. pKa du bromo-4 méthoxy-2 phénol : 9,5.

. pKa de l'acide conjugué de l'amine : 11,1

L'autoclave est fermé et purgé à l'aide d'un mélange équimoléculaire de CO et de $H_2$.

On charge ensuite 0,1 MPa (1 bar) de pression de ce mélange $CO/H_2$ ; on porte sous agitation le contenu de l'autoclave à 100°C ; on ajuste la pression $CO/H_2$ à 3 MPa (30 bar), puis la température est portée à 150°C.

On maintient la température à 150°C et la pression à 3 MPa jusqu'à la fin d'absorption du mélange $CO/H_2$.

L'autoclave est alors refroidi, dégazé.

On prélève un échantillon pour dosage par chromatographie liquide. Le traitement est celui qui est décrit pour les exemples 1 et 2.

Le tableau (II) ci-après reprend pour chaque essai les indications concernant la phosphine utilisée, la durée, le taux de transformation (TT %) du bromo-4 méthoxy-2 phénol (BMPH), le rendement (RT %) en hydroxy-4 méthoxy-3 benzaldéhyde (vanilline) (HMBZ) par rapport au bromo-4 méthoxy-2 phénol transformé, les RT % en gaïacol et éventuellement en acide hydroxy-4 méthoxy-3 benzoïque formés.

La différence à 100 % de la somme des RT indiqués précédemment correspond au composé polymérique indiqué dans les exemples 1 et 2.

On note que lorsque le pKa de la phosphine est inférieur à 5 le rendement en aldéhyde est nul (essai comparatif B).

TABLEAU II

| Essais | Phosphine | pKa * | Durée | TT % en BMPH ** | RT % en HMBZ ** | RT % en gaïacol | RT % en HMBQ ** |
|---|---|---|---|---|---|---|---|
| Exemple 3 | tricyclohexyl phosphine | 9,7 | 1 h 50 | 98 | 71 | 13 | 0,1 |
| Exemple 4 | triéthyl phosphine | 8,69 | 5 h 40 | 71 | 42 | 46,5 | 0 |
| Exemple 5 | tribenzyl phosphine | 6,0 | 3 h 25 | 84 | 69 | 28,5 | 0 |
| Essai comparatif B | triphényl phosphine | 2,74 | 20 min | 100 | 0 | 0 | 0 |

* pka de la phosphine

** BMPH = bromo-4 méthoxy-2 phénol
HMBZ = hydroxy-4 méthoxy-3 benzaldéhyde (vanilline)
HMBQ = acide hydroxy-4 méthoxy-3 benzoïque

Exemple 6 et essai comparatif C

Dans un autoclave de 125 $cm^3$, en alliage de marque commerciale HASTELLOY B2, muni d'un dispositif de chauffage et d'une agitation on charge :

- 8,65 (50 mmol) de bromo-2 phénol
- 0,22 (1 mmol) de diacétate de palladium
- 5 mmol de phosphine (indiquée dans le tableau ci-après)
- 110 mmol de triéthylamine
- 17,5 $cm^3$ de toluène.

- pKa du bromo-2 phénol : 8,55
- pKa de l'acide conjugué de l'amine : 11,1

L'autoclave est fermé et purgé à l'aide d'un mélange équimoléculaire de CO et de $H_2$.

On charge ensuite 0,1 MPa (1 bar) de pression de ce mélange $CO/H_2$ ; on porte sous agitation le contenu de l'autoclave à 100°C ; on ajuste la pression $CO/H_2$ à 3 MPa (30 bar), puis la température est portée à 150°C.

On maintient la température à 150°C et la pression à 3 MPa jusqu 'à la fin d'absorption du mélange $CO/H_2$.

L'autoclave est alors refroidi, dégazé.

On prélève un échantillon pour dosage par chromatographie liquide. Le traitement est celui qui est décrit pour les exemples 1 et 2.

Le tableau (III) ci-après reprend pour chaque essai les indications concernant la phosphine utilisée, la durée, le taux de transformation (TT %) du bromo-2 phénol (B2PH), le rendement (RT %) en hydroxy-2 benzaldéhyde ou aldéhyde salicylique (ALS) par rapport au bromo-2 phénol transformé, les RT % en phénol et éventuellement en acide salicylique (ACS) formés.

La différence à 100 % de la somme des RT indiqués précédemment correspond à un composé polymérique ayant une structure du type de celle indiquée dans les exemples 1 et 2.

On note que lorsque le pKa de la phosphine est inférieur à 5 le rendement en aldéhyde est faible (essai comparatif C).

TABLEAU III

| Essais | Phosphine | pKa * | Durée | TT % en B2PH ** | RT % en ALS ** | RT % en phénol | RT % en ACS ** |
|---|---|---|---|---|---|---|---|
| Exemple 6 | tricyclohexyl phosphine | 9,7 | 2 h 30 | 93 | 53 | 14 | 3 |
| Essai comparatif C | triphényl phosphine | 2,74 | 2 h 30 | 100 | 6 | 2 | 7 |

* pka de la phosphine

** B2PH = bromo-2 phénol
ALS = aldéhyde salicylique
ACS = acide salicylique

Exemple 7 et essai comparatif D

Dans un autoclave de 125 $cm^3$, en alliage de marque commerciale HASTELLOY B2, muni d'un dispositif de chauffage et d'une agitation on charge :

- 8,65 (50 mmol) de bromo-4 phénol
- 0,22 (1 mmol) de diacétate de palladium
- 5 mmol de phosphine (indiquée dans le tableau ci-après)
- 110 mmol de triéthylamine
- 17,5 $cm^3$ de toluène.
  . pKa du bromo-4 phénol : 9,5
  . pKa de l'acide conjugué de l'amine : 11,1

L'autoclave est fermé et purgé à l'aide d'un mélange équimoléculaire de CO et de $H_2$.

On charge ensuite 0,1 MPa (1 bar) de pression de ce mélange $CO/H_2$ ; on porte sous agitation le contenu de l'autoclave à 100°C ; on ajuste la pression $CO/H_2$ à 3 MPa (30 bar), puis la température est portée à 150°C.

On maintient la température à 150°C et la pression à 3 MPa jusqu'à la fin d'absorption du mélange $CO/H_2$.

L'autoclave est alors refroidi, dégazé.

On prélève un échantillon pour dosage par chromatographie liquide. Le traitement est celui qui est décrit pour les exemples 1 et 2.

Le tableau (IV) ci-après reprend pour chaque essai les indications concernant la phosphine utilisée, la durée, le taux de transformation (TT %) du bromo-4 phénol (B4PH), le rendement (RT %) en hydroxy-4 benzaldéhyde (H4BZ) par rapport au bromo-4 phénol transformé, les RT % en phénol et éventuellement en acide hydroxy-4 benzoïque (H4BQ) formés.

La différence à 100 % de la somme des RT indiqués précédemment correspond à un composé polymérique ayant une structure du type de celle indiquée dans les exemples 1 et 2.

On note que lorsque le pKa de la phosphine est inférieur à 5 le rendement en aldéhyde est très faible (essai comparatif D).

**TABLEAU IV**

| Essais | Phosphine | pKa * | Durée | TT % en B4PH ** | RT % en H4BZ ** | RT % en phénol | RT % en H4BQ ** |
|---|---|---|---|---|---|---|---|
| Exemple 7 | tricyclohexyl phosphine | 9,7 | 20 min | 92 | 58 | 9,8 | 2,2 |
| Essai comparatif D | triphényl phosphine | 2,74 | 20 min | 99 | 2 | 2 | 0 |

* pka de la phosphine

** B4PH = bromo-4 phénol
H4BZ = hydroxy-4 benzaldéhyde
H4BQ = acide hydroxy-4 benzoïque

## Revendications

1. Procédé de préparation d'un hydroxybenzaldéhyde de formule générale (I) :

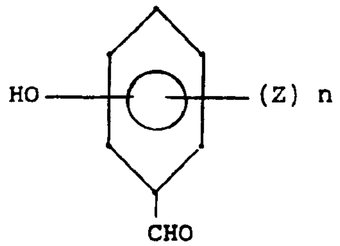

(I)

dans laquelle
- n représente 0, 1 ou 2
- Z représente un groupement électron-donneur ou un groupement électron-attracteur
par réaction d'un halogénophénol de formule générale (II)

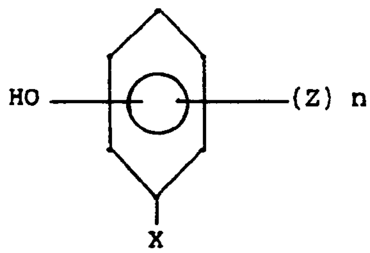

(II)

dans laquelle
- X représente un atome de brome ou un atome d'iode ;
- Z a les significations indiquées précédemment ;
- n représente 0, 1 ou 2 ;
avec un mélange monoxyde de carbone/hydrogène, en présence d'un catalyseur à base d'un métal noble, d'une amine tertiaire et d'une phosphine, caractérisé en ce que l'amine tertiaire est telle que le pKa de son acide conjugué soit supérieur au pKa de l'halogénophénol de formule (II) et, en ce que le pKa de la phosphine soit supérieur ou égal à 5.

2. Procédé selon la revendication 1 caractérisé en ce que l'on met en oeuvre un halogénophénol de formule (II) dans laquelle :
- le symbole Z représente un radical hydroxyle, un atome de brome, un atome d'iode, un radical alkyle, un radical alcoxy, un radical alkyle ou alcoxy substitué par un ou plusieurs atomes de chlore ou de fluor, un radical cycloalkyle, un radical phényle, un radical cycloalkoxy, un radical phénoxy, un radical alcoxycarbonyle, un radical cycloalcoxycarbonyle, un radical phénoxycarbonyle, un radical alkylcarbonyloxy, un radical cycloalkylcarbonyloxy, un radical phénylcarbonyloxy, l'un des radicaux précédents substitué par un ou plusieurs atomes de fluor et/ou de chlore ou groupes nitrile ;
- le symbole X représente un atome de brome ou un atome d'iode.

3. Procédé selon l'une des revendications 1 ou 2, caractérisé en ce que l'on met en oeuvre un halogénophénol de formule (II) dans laquelle :
- X représente un atome de brome,
- Z représente :
. un radical hydroxyle ;
. u atome de brome ;
. un radical alkyle linéaire ou ramifié de 1 à 20 atomes de carbone ou un radical alkyle substitué par un ou plusieurs atomes de fluor et/ou de chlore, tels que par exemple les radicaux méthyle, éthyle, propyles, butyles, pentyles, hexyles, octyles, décyles, trifluorométhyle, difluorochlorométhyle, trichlorométhyle ; de préférence un radical alkyle inférieur, c'est-à-dire ayant 1 à 4 atomes de carbone ou un radical alkyle inférieur substitué par 1 à 3 atomes de fluor et/ou de chlore ;
. un radical alkoxy linéaire ou ramifié ayant 1 à 20 atomes de carbone ou un radical alcoxy substitué par un ou plusieurs atomes de fluor et/ou de chlore ; de préférence un radical alcoxy inférieur (ayant 1 à 4 atomes de carbone) ou un radical alcoxy inférieur substitué par 1 à 3 atomes de fluor et/ou

de chlore, tel que par exemple les radicaux méthoxy, éthoxy, isopropoxy, difluorochlorométhoxy ou trichlorométhoxy ;

. un radical cyclopentyle, cyclohexyle ou cyclooctyle ;

. un radical phényle ou un radical phényle substitué par 1 à 3 radicaux alkyles ou alcoxy inférieurs, tel que les radicaux xylyles, toluyles, méthoxyphényles, éthoxyphényles ;

. un radical alcoxycarbonyle ayant 2 à 11 atomes de carbone et de préférence 2 à 5 atomes de carbone tels que par exemple les radicaux méthoxycarbonyle, éthoxycarbonyle, propoxycarbonyle et butoxycarbonyle ;

. un radical alcoxycarbonylalkyle dont la partie alcoxycarbonyle est telle que définie précédemment et la partie alkyle comporte 1 à 4 atomes de carbone ;

. un radical cyclopentyloxycarbonyle ou cyclohexyloxycarbonyle ;

. un radical phénoxycarbonyle ou méthylphénoxycarbonyle ;

. un radical alkylcarbonyloxy ayant 2 à 11 atomes de carbone et de préférence 2 à 5 atomes de carbone, tel que par exemple les radicaux acétoxy, propionyloxy, butyryloxy ;

. un radical cyclopentanoyloxy ou cyclohexanoyloxy ;

. un radical benzoyloxy, méthylbenzoyloxy ou diméthylbenzoyloxy.

**4.** Procédé selon l'une des revendications 1 à 3, caractérisé en ce que l'halogénophénol de formule (II) est le bromo-4 phénol,le bromo-2 phénol, le bromo-4 méthoxy-2 phénol, le bromo-2 méthoxy-4 phénol, le bromo-6 méthoxy-2 phénol, le bromo-4 éthoxy-2 phénol, le bromo-2 éthoxy-4 phénol, le bromo-6 éthoxy-2 phénol, le bromo-4 diméthoxy-2,6 phénol, le bromo-4 dihydroxy-1,2 benzène, le bromo-2 dihydroxy-1,4 benzène, le bromo-3 dihydroxy-1,2 benzène, le dibromo-2,4 phénol, le tribromo-2,4,6 phénol.

**5.** Procédé selon l'une des revendications 1 à 4, caractérisé en ce que le catalyseur utilisé est un métal noble finement divisé, du groupe VIII de la classification périodique des éléments tel que le palladium, le rhodium et l'iridium, ou leurs sels d'acides minéraux ou organiques.

**6.** Procédé selon l'une des revendications 1 à 5, caractérisé en ce que le catalyseur utilisé est choisi parmi les dérivés du palladium tels que les carboxylates, notamment les acétates, propionates, butyrates ou benzoates de palladium (II), et le chlorure palladeux.

**7.** Procédé selon l'une des revendications 1 à 6, caractérisé en ce que la quantité de catalyseur exprimée en moles d'atomes de métal ou en moles de dérivé métallique par mole d'halogènophénol de formule (I) est comprise entre $10^{-5}$ et $10^{-1}$ mole/mole et de préférence entre $10^{-4}$ et $10^{-2}$ mole/mole.

**8.** Procédé selon l'une des revendications 1 à 7, caractérisé en ce que la phosphine est une phosphine aliphatique, une phosphine cycloaliphatique, une phosphine arylaliphatique ou une phosphine mixte aliphatique et/ou cycloaliphatique et/ou arylaliphatique et/ou aromatique.

**9.** Procédé selon l'une des revendications 1 à 8, caractérisé en ce que la phosphine utilisée répond à la formule générale (III)

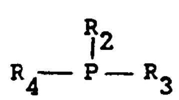 (III)

dans laquelle les symboles $R_2$, $R_3$ et $R_4$, identiques ou différents, représentent :

- un radical alkyle ayant de 1 à 12 atomes de carbone,
- un radical cycloalkyle ayant de 5 ou 6 atomes de carbone,
- un radical cycloalkyle ayant de 5 ou 6 atomes de carbone, substitué par un ou plusieurs radicaux alkyles ayant 1 à 4 atomes de carbone, alkoxy ayant 1 à 4 atomes de carbone,
- un radical phénylalkyle dont la partie aliphatique comporte de 1 à 6 atomes de carbone,
- un ou deux des radicaux $R_2$, $R_3$ et $R_4$ représentent un radical phényle ou un radical phényle substitué par un ou plusieurs radicaux alkyles ayant 1 à 4 atomes de carbone ou alkoxy ayant 1 à 4 atomes de carbone.

**10.** Procédé selon l'une des revendications 1 à 9, caractérisé en ce que la phosphine utilisée est choisie parmi la tricyclohexylphosphine, la triméthylphosphine, la triéthylphosphine, la tri-n-butylphosphine, la triisobutylphosphine, la tritertiobutylphosphine, la tribenzylphosphine, la dicyclohexylphénylphosphine, la diméthylphénylphosphine, la diéthylphénylphosphine, la ditertiobutylphénylphosphine.

**11.** Procédé selon l'une des revendications 1 à 10, caractérisé en ce que la quantité de phosphine libre et/ou sous forme de complexe avec le catalyseur, est telle que le rapport molaire phosphine/métal noble du catalyseur soit compris entre 2 et 10.000, et de préférence compris entre 4 et 1.000.

**12.** Procédé selon l'une des revendications 1 à 11, caractérisé en ce que l'amine tertiaire utilisée est une amine de formule générale (IV)

$$N - (R_1)_3 \qquad (IV)$$

dans laquelle :

- les radicaux $R_1$, identiques ou différents, représentent des restes hydrocarbonés ayant de 1 à 20 atomes de carbone, tels que des radicaux alkyle, cycloalkyle, aryle ou hétérocyclique ;
- 2 radicaux $R_1$ forment ensemble et avec l'atome d'azote un hétérocycle ayant 4 à 6 atomes.

**13.** Procédé selon la revendication 12, caractérisé en ce que l'amine tertiaire utilisée est une amine de formule générale (IV) dans laquelle

- les symboles $R_1$ représentent un radical alkyle ayant 1 à 10 atomes de carbone et de préférence 1 à 4 atomes de carbone ou un radical cyclopentyle ou cyclohexyle ou un radical pyridinyle ;
- 2 radicaux $R_1$ forment ensemble et avec l'atome d'azote un cycle pipéridine ou pyrrolidine.

**14.** Procédé selon les revendications 12 ou 13, caractérisé en ce que l'amine tertiaire est la triéthylamine, la tri-n-propylamine, la tri-n-butylamine, la méthyldibutylamine, la méthyldicyclohexylamine, l'éthyldiisopropylamine, la N,N-diéthylcyclohexylamine, la diméthylamino-4 pyridine, la N-méthylpipéridine, la N-éthylpipéridine, la N-n-butylpipéridine, la diméthyl-1,2 pipéridine, la N-méthylpyrrolidine, la diméthyl-1,2 pyrrolidine.

**15.** Procédé selon l'une des revendication 1 à 14, caractérisé en ce que la quantité d'amine tertiaire est suffisante pour neutraliser l'hydracide libéré par la réaction et en ce que de préférence la concentration en amine tertiaire dans le milieu est au moins égale à 2 moles par litre pendant la durée de la réaction.

**16.** Procédé selon l'une des revendications 1 à 15, caractérisé en ce que la pression utilisée est comprise entre 0,1 à 30 MPa (1 à 300 bar) et de préférence entre 1 à 15 MPa (10 à 150 bar).

**17.** Procédé selon l'une des revendications 1 à 16, caractérisé en ce qu'il est conduit dans un solvant inerte dans les conditions de la réaction d'hydrocarbonylation choisi parmi des hydrocarbures aliphatiques ou cycloaliphatiques saturés tels que l'hexane ou le cyclohexane, ou aromatiques tels que le benzène, le toluène, les xylènes ; des esters tels que le benzoate de méthyle, le téréphtalate de méthyle, l'adipate de méthyle, le phtalate de dibutyle ; des esters ou éthers de polyols tels que le diacétate de tétraéthylène glycol ; des éthers cycliques tels que le tétrahydrofuranne ou le dioxanne.

**18.** Procédé selon l'une des revendications 1 à 17, caractérisé en ce que la concentration de l'halogénophénol de formule (I) mis en oeuvre, exprimée en poids d'halogénophénol par volume de solvant, est comprise entre 5 % et 50 % et de préférence entre 10 % et 40 %.

**19.** Procédé selon l'une des revendications 1 à 18, caractérisé en ce qu'en fin d'essai le mélange réactionnel est traité par une solution aqueuse d'hydroxyde de métal alcalin.

## Patentansprüche

**1.** Verfahren zur Herstellung eines Hydroxybenzaldehyds der allgemeinen Formel (I):

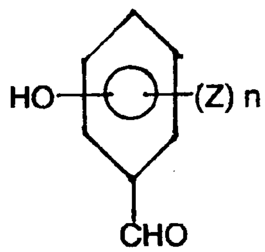

(I)

worin

n 0, 1 oder 2 und

Z eine elektronenspendende oder eine elektronenanziehende Gruppe bedeutet,

durch Reaktion eines Halogenphenols der allgemeinen Formel (II)

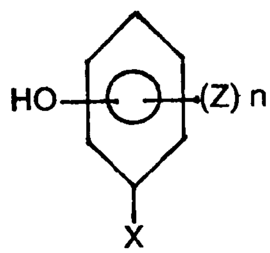

(II)

worin

X ein Brom- oder ein Jodatom bedeutet,

Z die oben angegebenen Bedeutungen hat,

n 0,1 oder 2 bedeutet;

und zwar mit einer Kohlenmonoxid/Wasserstoff-Mischung in Gegenwart eines Katalysators auf Edelmetallbasis, eines tertiären Amins und eines Phosphins, dadurch gekennzeichnet, daß das tertiäre Amin so beschaffen ist, daß der pka-Wert seiner konjugierten Säure über dem pKa-Wert des Halogenphenols der Formel (II) liegt und daß ferner der pka-Wert des Phosphins größer oder gleich 5 ist.

2. Verfahren nach Anspruch 1, dadurch gekennzeichnet, daß man ein Halogenphenol der Formel (II) einzetzt, in welcher:
   . Z ein Hydroxylrest, ein Bromatom, ein Jodatom, ein Alkylrest, ein Alkoxyrest, ein Alkyl- oder Alkoxyrest, der durch ein oder mehrere Chlor- oder Fluoratome substituiert ist, ein Cycloalkylrest, ein Phenylrest, ein Cycloalkoxyrest, ein Phenoxyrest, ein Alkoxycarbonylrest, ein Cycloalkoxycarbonylrest, ein Phenoxycarbonylrest, ein Alkylcarbonyloxyrest, ein Cycloalkylcarbonyloxyrest oder ein Phenylcarbonyloxyrest ist, wobei einer der vorgenannten Reste durch ein oder mehrere Fluor- und/oder Chloratome oder Nitrilgruppen substituiert ist; und
   . X ein Bromatom oder ein Jodatom bedeutet.

3. Verfahren nach einem der Ansprüche 1 oder 2, dadurch gekennzeichnet, daß man ein Halogenphenol der Formel (11) einsetzt, worin:
   . X ein Bromatom bedeutet, und
   . Z bedeutet:
      . einen Hydroxylrest;
      . ein Bromatom;
      . einen geradkettigen oder verzweigten Alkylrest mit 1 bis 20 Kohlenstoffatomen oder einen Alkylrest, der durch ein oder mehrere Fluor und/oder Chloratome substituiert ist, wie zum Beispiel einen Methyl-, Ethyl-, Propyl-, Butyl-, Pentyl-, Hexyl, Octyl-, Decyl-, Trifluormethyl-, Difluorchlormethyl-, Trichlormethylrest; vorzugsweise einen kurzkettigen Alkylrest mit 1 bis 4 Kohlenstoffatomen oder einen kurzkettigen Akylrest, der durch 1 bis 3 Fluor- und/oder Chloratome substituiert ist;
      . einen geradkettigen oder verzweigten Alkoyrest mit 1 bis 20 Kohlenstoffatomen oder einen Alkoxyrest, der durch ein oder mehrere Fluor- und/oder Chloratome substituiert ist; vorzugweise einen kurzkettigen Alkoxyrest (mit 1 bis 4 Kohlenstoffatomen) oder einen kurzkettigen Alkoxyrest, der

durch 1 bis 3 Fluor- und/oder Chloratome substituiert ist, wie zum Beispiel einen Methoxy-, Ethoxy-, Isopropoxy-, Difluorchlormethoxy- oder Trichlormethoxyrest;
. einen Cyclopentyl-, Cyclohexyl- oder Cyclooctylrest;
. einen Phenylrest oder einen Phenylrest, der durch 1 bis 3 kurzkettige Alkyl- oder Alkoxyreste substituiert ist, wie Xylyl-, Toluyl-, Methoxyphenyl-, Ethoxyphenylreste;
. einen Alkoxycarbonylrest mit 2 bis 11 Kohlenstoffatomen und vorzugsweise 2 bis 5 Kohlenstoffatomen wie zum Beispiel Methoxycarbonyl-, Ethoxycarbonyl-, Propoxycarbonyl- und Butoxycarbonylreste;
. einen Alkoxycarbonylalkylrest, dessen Alkoxycarbonylteil wie vorstehend definiert ist und dessen Alkylteil 1 bis 4 Kohlenstoffatome enthält;
. einen Cyclopentyloxycarbonyl- oder Cyclohexyloxycarbonylrest ;
. einen Phenoxycarbonyl- oder Methylphenoxycarbonylrest;
. einen Alkylcarbonyloxyrest mit 2 bis 11 Kohlenstoffatomen und vorzugweise 2 bis 5 Kohlenstoffatomen, wie zum Beispiel einen Acetoxy-, Propionyloxy-, Butyryloxyrest;
. einen Cyclopentanoyloxy- oder Cyclohexanoyloxyrest;
. einen Benzoyloxy-, Methylbenzoyloxy oder Dimethylbenzoyloxyrest.

**4.** Verfahren nach einem der Ansprüche 1 bis 3, dadurch gekennzeichnet, daß das Halogenphenol der Formel (II) Brom-4-phenol, Brom-2-phenol, Brom-4-methoxy-2-phenol, Brom-2-methoxy-4-phenol, Brom-6-methoxy-2-phenol,Brom-4-ethoxy-2-phenol, Brom-2-Ethoxy-4-phenol, Brom-6-ethoxy-2-phenol, Brom-4-dimethoxy-2,6-phenol, Brom4-dihydroxy-1,2-benzol, Brom-2-dihydroxy-1,4-benzol, Brom-3-dihydroxy-1,2-benzol, Dibrom-2,4-phenol oder Tri-brom-2,4,6-phenol ist.

**5.** Verfahren nach einem der Ansprüche 1 bis 4, dadurch gekennzeichnet, daß der verwendete Katalysator aus einem feinverteilten Edelmetall der Gruppe VIII des Periodensystems der Elemente ist, wie zum Beispiel Palladium, Rhodium und Iridium oder ihren Salzen von Mineralsäuren oder organischen Säuren.

**6.** Verfahren nach einem der Ansprüche 1 bis 5 dadurch gekennzeichnet, daß der verwendete Katalysator aus Palladiumderivaten ausgewählt wird, wie Carboxylaten, insbesonder Palladium(II)-acetan, -propionaten, -butyraten oder -benzoaten und Palladium(II)-chlorid.

**7.** Verfahren nach einem der Ansprüche 1 bis 6, dadurch gekennzeichnet, daß die Katalysatormenge ausgedrückt in Mol Metallatome oder in Mol Metallderivate je Mol Halogenphenol der formel (I) zwischen $10^{-5}$ und $10^{-1}$ Mol/Mol, vorzugsweise zwischen $10^{-4}$ und $10^{-2}$ Mol/Mol liegt.

**8.** Verfahren nach einem der Ansprüche 1 bis 7, dadurch gennzeichnet, daß das Phosphin ein aliphatisches Phosphin, ein cycloaliphatisches Phosphin, ein arylaliphatisches Phosphin oder ein gemischt aliphatisches und/oder cycloaliphatisches und/oder arylaliphatisches und/oder aromatisches Phosphin ist.

**9.** Verfahren nach einem der Ansprüche 1 bis 8, dadurch gekennzeichnet, daß das verwendete Phosphin der allgemeinen Formen (III) entspricht:

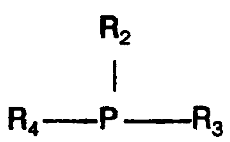

(III)

worin $R_2$, $R_3$ und $R_4$ identisch oder unterschiedlich sind und darstellen:
. einen Alkylrest mit 1 bis 12 Kohlenstoffatomen,
. einen Cycloalkylrest mit 5 oder 6 Kohlenstoffatomen,
. einen Cycloalkylrest mit 5 oder 6 Kohlenstoffatomen, der durch ein oder mehrere Alkylreste mit 1 bis 4 Kohlenstoffatomen, Alkoxyreste mit 1 bis 4 Kohlenstoffatomen substituiert ist,
. einen Phenylalkylrest, dessen aliphatischer Teil 1 bis 6 Kohlenstoffatome enthält,
. ein oder zwei der Reste $R_2$, $R_3$ und $R_4$ bedeuten einen Phenylrest oder einen Phenylrest, der durch ein oder mehrere Alkylreste mit 1 bis 4 Kohlenstoffatomen oder Alkoxyresten mit 1 bis 4 Kohlenstoffatomen substituiert ist.

**10.** Verfahren nach einem der Ansprüche 1 bis 9, dadurch gekennzeichnet, daß das verwendete Phosphin aus folgenden ausgewählt wird: Tricyclohexylphosphin, Trimethylphosphin, Triethylphosphin, Tri-n-butylphosphin, Triisobutylkphosphin, Tritertiobutylphosphin, Tribenzylphosphin, Dicyclohexylphenylphosphin, Dimethylphenylphosphin, Diethylphenylphosphin, Ditertiärbutylphenylphosphin.

**11.** Verfahren nach einem der Ansprüche 1 bis 10, dadurch gekennzeichnet, daß die Menge freien Phosphins und/oder Phosphins in Form eines Komplexes mit dem Katalysator so ist, daß das Molverhältnis Phosphin/Katalysatoredelmetall zwischen 2 und 10.000, vorzugsweise zwischen 4 und 1.000 liegt.

**12.** Verfahren nach einem der Ansprüche 1 bis 11, dadurch gekennzeichnet, daß das verwendete tertiäre Amin ein Amin der allgemeinen Formel (IV):

$$N\text{-}(R_1)_3 \qquad (IV)$$

ist, worin

. die Reste $R_1$ identisch oder unterschiedlich sind und Kohlenwasserstoffreste mit 1 bis 20 Kohlenstoffatomen, wie Alkyl-, Cycloalkyl-, Aryl- oder heterocyclische Reste bedeuten; und

. 2 Reste $R_1$ zusammen und mit dem Stickstoffatom eine heterocyclische Verbindung mit 4 bis 6 Atomen bilden.

**13.** Verfahren nach Anpruch 12, dadurch gekennzeichnet, daß das verwendete tertiäre Amin ein Amin der allgemeinen Formel (IV) ist, worin

. $R_1$ ein Alkylrest mit 1 bis 10 Kohlenstoffatomen und vorzugsweise 1 bis 4 Kohlenstoffatomen oder ein Cyclopentyl- oder Cyclohexylrest oder ein Pyridinylrest ist; und

. 2 Reste $R_1$ zusammen und mit dem Stickstoffatom einen Piperidin-oder Pyrrolidinring bilden.

**14.** Verfahren nach den Ansprüchen 12 oder 13, dadurch gekennzeichnet, daß das tertiäre Amin Triethylamin, Tri-n-propylamin, Tri-n-butylamin, Methyldibutylamin, Methyldicyclohexylamin, Ethyldiisopropylamin, N,N-diethylcyclohexylamin, Dimethylamino-4-pyridin, N-methylpiperidin, N-ethylpiperidin, N-n-butylpiperidin, Dimethyl-1,2-piperidin, N-methylpyrrolidin oder Dimethyl-1,2-pyrrolidin ist.

**15.** Verfahren nach einem der Ansprüche 1 bis 14, dadurch gekennzeichnet, daß die Menge tertiären Amins ausreicht, um die durch die Reaktion freigesetzte Wasserstoffsäure zu neutraliesieren und daß die konzentration des tertiären Amins in dem Medium während der Reaktionsdauer vorzugsweise mindestens gleich 2 Mol je Liter beträgt.

**16.** Verfahren nach eienem der Ansprüche 1 bis 15, dadurch gekennzeichnet, daß der verwendete Druck zwischen 0,1 bis 30 MPa (1 bis 300 bar) und vorzugsweise zwischen 1 bis 15 MPa (10 bis 150 bar) beträgt.

**17.** Verfahren nach einem der Ansprüche 1 bis 16, dadurch gekennzeichnet, daß es in einem inerten Lösungsmittel unter den Bedingungen einer Hydrocarbonylierungsreaktion durchgeführt wird, wobei das Lösungsmittel aus gewählt wird aus: aliphatischen oder cycloaliphatischen gesättigten Kohlenwasserstoffen wie Hexan oder Cyclohexan, oder aromatischen Kohlenwasserstoffen wie Benzol, Toluol, Xylolen; Estern wie Methylbenzoat, Methylterephtalat, Methyladipat, Dibutylphtalat; Polyolestern oder -ethern wie Tetraethylenglycoldiacetat; cyclischen Esthern wie Tetrahydrofuran oder Dioxan.

**18.** Verfahren nach einem der Ansprüche 1 bis 17, dadurch gekennzeichnet, daß die Konzentration des eingesetzten Halogenphenols der Formel (I) ausgedrückt in Halogenphenolgewicht je Lösungsmittelvolumen zwischen 5% und 50% und vorzugsweise zwischen 10% und 40% beträgt.

**19.** Verfahren nach einem der Ansprüche 1 bis 18, dadurch gekennzeichnet, daß das Reaktionsgemisch am Ende des Versuches mit einer wässrigen Alkalimetallhydroxidlösung behandelt wird.

## Claims

**1.** Process for the preparation of a hydroxy-benzaldehyde of general formula (I):

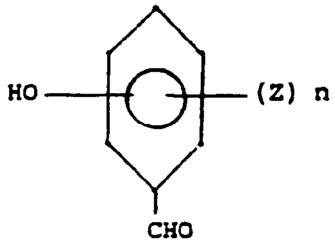

(I)

in which

- n denotes 0, 1 or 2
- Z denotes an electron-donating group or an electron-attracting group

by reaction of a halophenol of general formula (II)

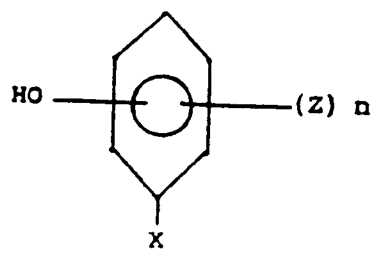

(II)

in which

- X denotes a bromine atom or an iodine atom;
- Z has the meanings shown above;
- n denotes 0, 1 or 2;

with a carbon monoxide/hydrogen mixture in the presence of a catalyst based on a noble metal, of a tertiary amine and of a phosphine, characterised in that the tertiary amine is such that the $pK_a$ of its conjugate acid is higher than the $pK_a$ of the halophenol of formula (II) and in that the $pK_a$ of the phosphine is higher than or equal to 5.

2. Process according to claim 1, characterised in that a halophenol of formula (II) is employed, in which:
   - the symbol Z denotes a hydroxyl radical, a bromine atom, an iodine atom, an alkyl radical, an alkoxy radical, an alkyl or alkoxy radical substituted by one or more chlorine or fluorine atoms, a cycloalkyl radical, a phenyl radical, a cycloalkoxy radical, a phenoxy radical, an alkoxycarbonyl radical, a cycloalkoxycarbonyl radical, a phenoxy carbonyl radical, an alkylcarbonyloxy radical, a cycloalkylcarbonyloxy radical, a phenylcarbonyloxy radical, one of the above radicals substituted by one or more fluorine and/or chlorine atoms or nitrile groups;
   - the symbol X denotes a bromine atom or an iodine atom.

3. Process according to either of claims 1 or 2, characterised in that a halophenol of formula (II) is employed, in which:
   - X denotes a bromine atom,
   - Z denotes:

     a hydroxyl radical;

     a bromine atom;

     a linear or branched alkyl radical containing 1 to 20 carbon atoms or an alkyl radical substituted by one or more fluorine and/or chlorine atoms, such as, for example, methyl, ethyl, propyl, butyl, pentyl, hexyl, octyl, decyl, trifluoromethyl, difluorochloromethyl and trichloromethyl radicals; preferably a lower alkyl radical, that is to say containing 1 to 4 carbon atoms, or a lower alkyl radical substituted by 1 to 3 fluorine and/or chlorine atoms;

     a linear or branched alkoxy radical containing 1 to 20 carbon atoms or an alkoxy radical substituted by one or more fluorine and/or chlorine atoms; preferably a lower alkoxy radical (containing 1 to 4 carbon atoms) or a lower alkoxy radical substituted by 1 to 3 fluorine and/or chlorine atoms, such as, for example, methoxy, ethoxy, isopropoxy, difluorochloromethoxy, or trichloromethoxy radicals; a cyclopentyl, cyclohexyl or cyclooctyl radical;

a phenyl radical or a phenyl radical substituted by 1 to 3 lower alkyl or alkoxy radicals, such as xylyl, tolyl, methoxyphenyl or ethoxyphenyl radicals;

an alkoxycarbonyl radical containing 2 to 11 carbon atoms and preferably 2 to 5 carbon atoms, such as, for example, methoxycarbonyl, ethoxycarbonyl, propoxycarbonyl and butoxycarbonyl radicals;

an alkoxycarbonylalkyl radical in which the alkoxycarbonyl part is such as defined above and the alkyl part contains 1 to 4 carbon atoms;

a cyclopentyloxycarbonyl or cyclohexyloxycarbonyl radical;

a phenoxycarbonyl or methylphenoxycarbonyl radical;

an alkylcarbonyloxy radical containing 2 to 11 carbon atoms and preferably 2 to 5 carbon atoms, such as, for example, acetoxy, propionyloxy or butyryloxy radicals;

a cyclopentanoyloxy or cyclohexanoyloxy radical;

a benzoyloxy, methylbenzoyloxy or dimethylbenzoyloxy radical.

**4.** Process according to one of claims 1 to 3, characterised in that the halophenol of formula (II) is 4-bromophenol, 2-bromophenol, 4-bromo-2-methoxyphenol, 2-bromo-4-methoxyphenol, 6-bromo-2-methoxyphenol, 4-bromo-2-ethoxyphenol, 2-bromo-4-ethoxyphenol, 6-bromo-2-ethoxyphenol, 4-bromo-2,6-dimethoxyphenol, 4-bromo-1,2-dihydroxybenzene, 2-bromo-1,4-dihydroxybenzene, 3-bromo-1,2-dihydroxybenzene, 2,4-dibromophenol and 2,4,6-tri-bromophenol.

**5.** Process according to one of claims 1 to 4, characterised in that the catalyst employed is a finely divided noble metal of group VIII of the Periodic Classification of the elements, such as palladium, rhodium and iridium, or their inorganic or organic acid salts.

**6.** Process according to one of claims 1 to 5, characterised in that the catalyst employed is chosen from palladium derivatives such as the carboxylates, especially palladium(II) acetates, propionates, butyrates or benzoates, and palladous chloride.

**7.** Process according to one of claims 1 to 6, characterised in that the quantity of catalyst, expressed in moles of metal atoms or in moles of metal derivative per mole of halophenol of formula (I) is between $10^{-5}$ and $10^{-1}$ mole/mole and preferably between $10^{-4}$ and $10^{-2}$ mole/mole.

**8.** Process according to one of claims 1 to 7, characterised in that the phosphine is an aliphatic phosphine, a cycloaliphatic phosphine, an arylaliphatic phosphine or a mixed aliphatic and/or cycloaliphatic and/or arylaliphatic and/or aromatic phosphine.

**9.** Process according to one of claims 1 to 8, characterised in that the phosphine employed corresponds to the general formula (III)

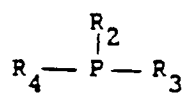 (III)

in which the symbols $R_2$, $R_3$ and $R_4$, which are identical or different, denote:

- an alkyl radical containing from 1 to 12 carbon atoms,
- a cycloalkyl radical containing 5 or 6 carbon atoms,
- a cycloalkyl radical containing 5 or 6 carbon atoms, substituted by one or more alkyl radicals containing 1 to 4 carbon atoms, or alkoxy radicals containing 1 to 4 carbon atoms,
- a phenylalkyl radical in which the aliphatic part contains from 1 to 6 carbon atoms,
- one or two of the radicals $R_2$, $R_3$ and $R_4$ denote a phenyl radical or a phenyl radical substituted by one or more alkyl radicals containing 1 to 4 carbon atoms or alkoxy radicals containing 1 to 4 carbon atoms.

**10.** Process according to one of Claims 1 to 9, characterised in that the phosphine employed is chosen from tricyclohexylphosphine, trimethylphosphine, triethylphosphine, tri-n-butylphosphine, triisobutylphosphine, tri-tert-butylphosphine, tribenzylphosphine, dicyclohexylphenylphosphine, dimethylphenylphosphine, diethylphenylphosphine and di-tert-butylphenylphosphine.

**11.** Process according to one of claims 1 to 10, characterised in that the quantity of phosphine which is free and/or in the form of a complex with the catalyst is such that the molar ratio phosphine/noble metal of the catalyst is between 2 and 10,000, and preferably between 4 and 1,000.

**12.** Process according to one of claims 1 to 11, characterised in that the tertiary amine employed is an amine of general formula (IV)

$$N - (R_1)_3 \qquad (IV)$$

in which:

- the radicals $R_1$, which are identical or different, denote hydrocarbon residues containing from 1 to 20 carbon atoms, such as alkyl, cycloalkyl, al or heterocyclic radicals;
- 2 radicals $R_1$ form together and with the nitrogen atom a heterocyclic ring containing 4 to 6 atoms.

**13.** Process according to claim 12, characterised in that the tertiary amine employed is an amine of general formula (IV) in which

- the symbols $R_1$ denote an alkyl radical containing 1 to 10 carbon atoms and preferably 1 to 4 carbon atoms or a cyclopentyl or cyclohexyl radical or a pyridinyl radical;
- 2 radicals $R_1$ form together and with the nitrogen atom a piperidine or pyrrolidine ring.

**14.** Process according to claims 12 of 13, characterised in that the tertiary amine is triethylamine, tri-n-propylamine, tri-n-butylamine, methyldibutylamine, methyldicyclohexylamine, ethyldiisopropylamine, N,N-diethylcyclohexylamine, 4-dimethylaminopyridine, N-methylpiperidine, N-ethylpiperidine,N-n-butylpiperidine, 1,2-dimethylpiperidine, N-methylpyrrolidine and 1,2-dimethylpyrrolidine.

**15.** Process according to one of claims 1 to 14, characterised in that the quantity of tertiary amine is sufficient to neutralise the hydracid released by the reaction and that the concentration of tertiary amine in the medium is preferably at least equal to 2 moles per litre during the reaction period.

**16.** Process according to one of claims 1 to 15, characterised in that the pressure employed is between 0.1 and 30 MPa (1 to 300 bar) and preferably between 1 and 15 MPa (10 to 150 bar).

**17.** Process according to one of claims 1 to 16, characterised in that it is conducted in a solvent which is inert under the conditions of the hydrocarbonylation reaction, chosen from saturated aliphatic or cycloaliphatic hydrocarbons such as hexane or cyclohexane, or aromatic hydrocarbons such as benzene, toluene and xylenes; esters such as methyl benzoate, methyl terephthalate, methyl adipate and dibutyl phthalate, polyol esters or ethers such as tetraethylene glycol diacetate; and cyclic ethers such as tetrahydrofuran or dioxane.

**18.** Process according to one of claims 1 to 17, characterised in that the concentration of the halophenol of formula (I) employed, expressed as weight of halophenol per volume of solvent, is between 5 % and 50 %, and preferably between 10 % and 40 %.

**19.** Process according to one of claims 1 to 18, characterised in that at the end of a test the reaction mixture is treated with an aqueous solution of an alkali metal hydroxide.